# EUROPEAN PATENT SPECIFICATION

(11) **EP 2 558 296 B1**
(45) Date of publication and mention of the grant of the patent: **02.07.2014**
(21) Application number: 11714471.7
(22) Date of filing: 01.04.2011
(51) Int. Cl.: B32B 37/15, A44B 18/00, A61B 5/022, A61F 5/44, B29C 65/74, B32B 38/06

(54) **METHOD OF FORMING LAMINATES FOR TOUCH FASTENERS**
VERFAHREN ZUR HERSTELLUNG VON VERBUNDSTOFFEN
PROCÉDÉ DE FORMATION DE MATÉRIAUX COMPOSITES

(30) Priority: 15.04.2010 US 324545 P
(43) Date of publication of application: 20.02.2013
(73) Proprietor: Velcro Industries B.V., Curacao (NL)
(72) Inventor: SHEPARD, William H., Amherst, New Hampshire 03031 (US)
(74) Representative: Schley, Jan Malte
(86) International application number: PCT/US2011/030887
(87) International publication number: WO 2011/130020

(56) References cited:
- WO-A1-02/096233
- WO-A2-01/91593
- US-B1- 6 202 260

## Description

### TECHNICAL FIELD

This invention relates to methods of forming composite materials.

### BACKGROUND

Multi-layered composite materials are used for various different types of applications. In certain cases, such composite materials are constructed in a way to make the composite materials substantially liquid-impermeable and/or air-impermeable. Liquid-impermeable and/or air-impermeable composite materials can be used in products in which it is useful to prevent liquid and/or air from escaping from the product during use.

WO 02/096233 A1 discloses a fastener product that includes a longitudinally extending strip-form base and a plurality of longitudinally discrete regions of fastener element stems. Each discrete region includes a thermoplastic resin layer bonded to a second base material by an intermingling of the resin of the resin layer with the second base material and the fastener element stems are molded, integral extensions of the resin from the resin layer. Various methods of making such a fastener product and others, include introducing various barrier and backing materials into the molding gap to limit the fastener element stem formation and/or the lamination of the resin to the barrier and/or backing material during the molding process.

### SUMMARY

In one aspect of the invention, a method of forming a composite material includes introducing a sheet-form film and a hook-engageable material into a nip formed between a first roll and a second roll, introducing molten resin into the nip between the film and the hook-engageable material, and allowing the molten resin to cool so that the film becomes attached to the hook-engageable material by the cooled resin. Each of the film, the hook-engageable material, and the molten resin includes at least about 85 percent by weight of a first polymer.

In another aspect of the invention, a method of forming a composite material includes introducing a sheet-form film and a hook-engageable material into a nip formed between a first roll and a second roll, introducing molten resin into the nip between the film and the hook-engageable material, and allowing the molten resin to cool so that the film becomes attached to the hook-engageable material by the cooled resin. Each of the film, the hook-engageable material, and the molten resin includes at least about 90 percent by weight of a first polymer, the composite material is substantially air-impermeable, and fibers extending from a surface of the hook-engageable material are capable of engaging hooks of hook fasteners after the film is attached to the hook-engageable material by the cooled resin.

Embodiments can include one or more of the following features.

In some embodiments, the composite material is substantially air-impermeable. In certain embodiments, the composite material has an air-permeability of 0 cm3/s/cm2.

In some embodiments, fibers extending from a surface of the hook-engageable material are capable of engaging hooks of hook fasteners after the film is attached to the hook-engageable material by the cooled resin.

In certain embodiments, the first polymer is polypropylene.

In some embodiments, the film and the hook-engageable material consist essentially of polypropylene.

In certain embodiments, the molten resin is at least about 90 percent by weight polypropylene.

In some embodiments, the molten resin further includes polyethylene.

In certain embodiments, the hook-engageable material includes a 17 grams per square meter spunbond polypropylene substrate through which a plurality of loop shaped staple fibers extend.

In some embodiments, the hook-engageable material includes a 30 grams per square meter SMS polypropylene substrate through which a plurality of loop shaped staple fibers extend.

In certain embodiments, the molten resin introduced into the nip has a thickness of about 0,0254 mm (one mil), the film has a thickness of about 0,0762 mm (three mils), and the hook-engageable material has a weight of about 44.1 g/m² to about 67.8 g/m² (about 1.3 to about 2.0 osy).

In some embodiments, the hook-engageable material has a basis weight of about 44.1 g/m² to about 54.2 g/m² (about 1.3 to about 1.6 osy).

In certain embodiments, the molten resin is at a temperature of at least about 260 degrees celsius (500 degrees Fahrenheit) (e.g., about 287.8 degrees celsius to about 315.6 degrees celsius (550 degrees Fahrenheit to about 600 degrees Fahrenheit), about 304.4 degrees celsius (about 580 degrees Fahrenheit)) when introduced into the nip.

In some embodiments, the film is a cast film.

In certain embodiments, the molten resin is introduced into the nip in a manner such that the molten resin contacts the film and the hook-engageable material at substantially the same time.

In some embodiments, the molten resin is introduced into the nip by applying the molten resin to the hook-engageable material and rotating the first and second rolls to carry the film, the hook-engageable material, and the molten resin into the nip.

In certain embodiments, the molten resin is introduced into the nip by applying the molten resin to the film and rotating the first and second rolls to carry the film, the hook-engageable material, and the molten resin into the nip.

In some embodiments, at least one of the first and second rolls is chilled to facilitate cooling of the molten resin.

In certain embodiments, both of the first and second rolls are chilled.

In some embodiments, the method further includes embossing the composite material.

In certain embodiments, embossing the composite material includes passing the composite material between an embossing roll and a backing roll. The embossing roll has a plurality of raised features that compress the composite material between the embossing roll and the backing roll.

In some embodiments, the embossing roll is a heated roll.

Embodiments can include one or more of the following advantages.

In certain embodiments, the composite materials formed using the various methods described herein are formed substantially entirely of a single type of polymer (e.g., polypropylene). Each component or layer of the composite material can, for example, include at least about 85 percent by weight (e.g., at least about 90 percent by weight, at least about 95 percent by weight) of the single type of polymer. Such composite materials can, for example, be easily recycled. In addition, such composite materials can provide improved weldability. For example, because the various components of the composite material are formed of substantially entirely the same type of material, it is relatively easy to thermally bond those components together.

In some embodiments, the composite materials formed using the various methods described herein are air-impermeable. As a result, the composite materials can be used to form inflatable bladders (e.g., inflatable bladders used in medical products, such as inflatable compression devices). The composite materials can alternatively or additionally be substantially liquid-impermeable, which allows the composite materials to be used as liquid barriers. Such liquid barriers can be advantageously used in products, such as medical cold wraps, where it is desirable to prevent a freezable cooling solution or water from melting and leaking out of the product.

In certain embodiments, the composite materials formed using the various methods described herein include a hook-engageable surface. In such embodiments, the composite materials can be used to form products that benefit from being releasably fastened. Examples of such products include medical wraps and compression devices.

In some embodiments, the composite material is embossed before being formed into or incorporated into a product. It has been found that the embossed regions of the composite material facilitate folding or bending of the composite material. When inflatable products, such as medical compression devices, are formed using the embossed composite material, the noise associated with inflating the product can be reduced as a result of the embossed composite material. For example, crinkling noises often associated with the inflation of relatively thin materials can be reduced due to the facilitated folding or bending of the composite material along the embossed regions. This can be particularly advantageous for blood pressure cuffs used with blood pressure monitoring devices that detect audible signals while inflating and deflating the cuff.

Certain methods described herein can be used to rapidly and efficiently produce composite materials that are formed substantially entirely of a single type of polymer (e.g., polypropylene), that are substantially air-impermeable and/or liquid impermeable, and that include a hook-engageable surface. It has been discovered, for example, that sufficient amounts of molten resin polymer can be applied between a polymeric film and polymeric loop material to achieve a substantially air-impermeable and/or liquid-impermeable composite material without destroying the functionality of the loops of the loop material, even when the molten resin and the loop material are formed substantially entirely of the same type of polymeric material (e.g., even when at least about 85 percent by weight of the molten resin and at least about 85 percent by weight of the loop material is the same type of polymeric material, such as polypropylene) and thus have similar melting temperatures.

Other aspects, features, and advantages will be apparent from the description and drawings, and from the claims.

### DESCRIPTION OF DRAWINGS

Fig. 1 is a schematic of a lamination system during use to form a substantially air-impermeable composite material.
Fig. 2 is an enlarged view of region 2 in Fig. 1, showing loops, which are capable of engaging hooks of hook fastener products, extending from a loop material layer of the composite material.
Fig. 3 is an enlarged view of region 3 in Fig. 1, showing the multiple bonded layers of the composite material.
Figs. 4 and 5 are front and side views, respectively, of a blood pressure cuff manufactured in part from substantially air-impermeable composite material formed using the method schematically illustrated in Fig. 1.
Fig. 6 illustrates the blood pressure cuff of Figs. 4 and 5 during use.
Fig. 7 illustrates a method of manufacturing multiple blood pressure cuffs of the type shown in Figs. 4-6 by folding a single sheet of the composite material of Fig. 1 and then welding overlapping regions of the folded sheet together.
Fig. 8 illustrates an alternative method of manufacturing multiple blood pressure cuffs of the type shown in Figs. 4-6 by welding two sheets of the composite material of Fig. 1 together.
Fig. 9 is a front view of a sequential compression device manufactured in part from substantially air-impermeable composite material formed using the method schematically illustrated in Fig. 1.
Fig. 10 illustrates the sequential compression device of Fig. 9 configured for use.
Fig. 11 is a perspective view of a urinary drain bag.

### DETAILED DESCRIPTION

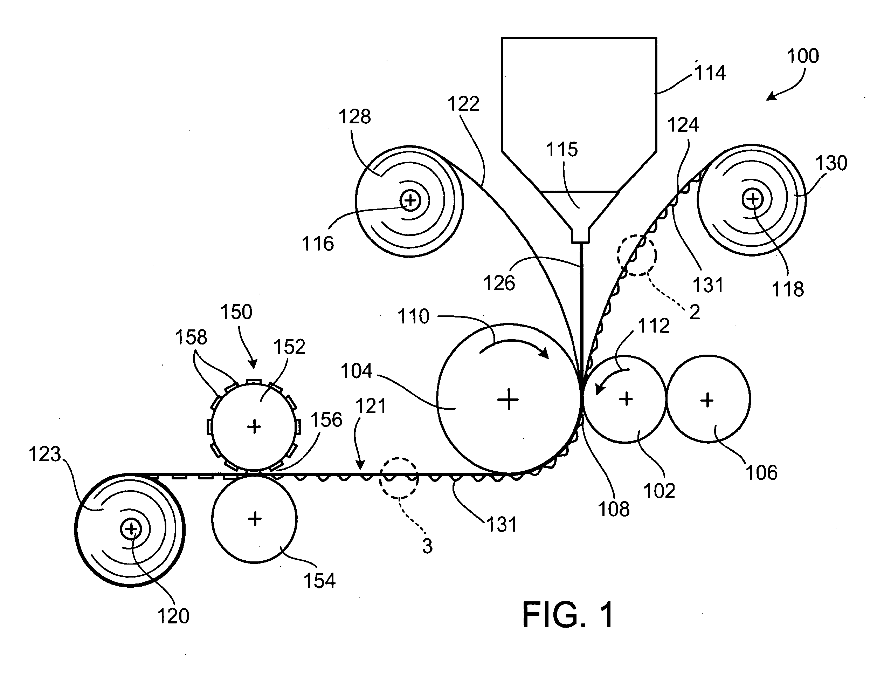

Fig. 1 schematically illustrates a lamination system 100 during use. As shown in Fig. 1, system 100 includes a pressure roll 102 and two backing rolls 104 and 106. Rolls 102 and 104 are positioned adjacent one another and form a nip 108 between their peripheral surfaces. Pressure roll 102 and backing roll 104 are connected to motors that are configured to rotate rolls 102 and 104 in opposite directions during use, as indicated by arrows 110 and 112. When operated in this manner, pressure roll 102 and backing roll 104 can cooperate to draw material (e.g., film, loop material, and/or resin) through nip 108 while applying pressure to the material passing through nip 108.

Backing roll 106 is positioned adjacent pressure roll 102 on the opposite side of pressure roll 102 from backing roll 104. Backing roll 106 is equipped with bearings such that backing roll 106 can rotate in response to the rotation of pressure roll 102. Backing roll 106 serves as a support for pressure roll 102 to help prevent pressure roll 102 from bowing outward as a result of increased pressure within nip 108. As a result, backing roll 106 helps to ensure that a desired uniform pressure is maintained in nip 108 during use.

Pressure roll 102 is a steel roll coated with polytetrafluoroethylene (PTFE). Typically, pressure roll 102 has a diameter of about 18 inches to about 24 inches. Backing rolls 104 and 106 are formed of steel and are uncoated. Each of rolls 102, 104, and 106 is capable of being chilled. For example, these rolls can included cooling passages through which a cooling liquid, such as water, can travel during use. The cooling liquid can be forced through the passages continuously during use to maintain the rolls at a reduced temperature.

A slot die extruder 114 is positioned above nip 108. Extruder 114 includes a die 115 through which molten resin is extruded during use. Extruder 114 is adjustable both vertically and horizontally such that extruder die 115 can be positioned at multiple different locations relative to nip 108.

Two rotatable shafts 116 and 118 are positioned above rolls 102 and 104. Rotatable shafts 116 and 118 are connected to motors that can drive shafts 116 and 118 at multiple different rotational speeds. As shown in Fig. 1, shaft 116 is configured such that a film roll 128 can be loaded thereon, and shaft 118 is configured such that a loop material roll 130 can be loaded thereon. As described below, during use, film 122 from film roll 128 and loop material 124 from loop material roll 130 can be introduced into nip 108 by rotating shafts 116 and 118, causing the rolls 128, 130 to unwind.

System 100 also includes another rotatable shaft referred to as a winder 120. Winder 120, like shafts 116 and 118, is connected to a motor that can drive winder 120 at multiple different rotational speeds. As described below, a substantially air-impermeable composite material 121 exiting nip 108 can be wound onto winder 120 for storage, shipment, or further processing by rotating winder 120. The rotational speeds of rolls 102, 104, 106, shafts 116, 118, and winder 120 can be controlled so that a desired tension is applied to the composite material exiting nip 108 and being wound onto winder 120.

Still referring to Fig. 1, an exemplary method of manufacturing substantially air-impermeable composite material 121 will now be described. During the manufacturing process, sheet-form film 122, loop material 124, and molten resin 126 are delivered to nip 108. Film 122 and loop material 124 are delivered to nip 108 from rolls 128 and 130 by rotating shafts 116 and 118 on which those rolls are mounted, and molten resin 126 is extruded into nip 108 from extruder 114. In the exemplary method, film 122, loop material 124, and molten resin 126 are formed substantially entirely of the same type of material. In particular, each of those components is formed substantially entirely of polypropylene. As discussed below, one or more of these components may include a polymer or polymers different from polypropylene, but the vast majority (e.g., at least about 85 percent by weight) of the materials used to form these components is polypropylene.

The melting temperatures of the materials from which film 122, loop material 124, and molten resin 126 are formed typically differ from one another by no more than about 25 degrees Fahrenheit (e.g., no more than about 20 degrees Fahrenheit, no more than about 15 degrees Fahrenheit, no more than about 10 degrees Fahrenheit). As discussed above, film 122, loop material 124, and molten resin 126 each include a substantial amount of polypropylene. Thus, substantial portions of the material from which these components are formed will have similar (e.g., substantially the same) melting temperatures. As a result of the similar melting temperatures of these materials, it can be quite complicated to bond molten resin 126 to loop material 124 without melting the loops of loop material 124. However, as discussed below, it has been found that this can be accomplished by using manufacturing process parameters and materials described herein.

Film 122 is a sheet-form polypropylene cast film having a thickness of about 0,0254 mm (one mil) to about 0.127 mm (five mils) (e.g., about 0.0762 mm (three mils)). The use of film 122 in combination with molten resin 126 helps to ensure that composite material 121 is substantially air-impermeable.

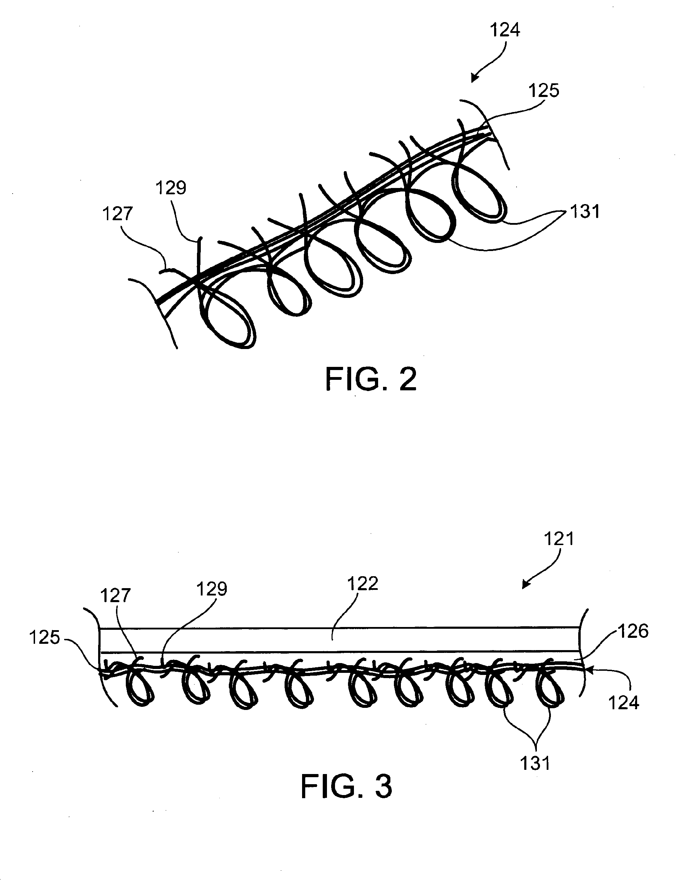

Referring briefly to Fig. 2, which is an enlarged view of region 2 in Fig. 1, loop material 124 includes a spunbond polypropylene substrate 125 having polypropylene staple fibers 127 and 129 needled therethrough to form loops 131 that extend from one side of substrate 125. Substrate 125 has a basis weight of about 17 grams per square meter. Staple fibers 127 and 129 are provided on substrate 125 in a 4:1 ratio. Staple fibers 127 are available from Asota Mills, under product number L10D. Staple fibers 129 are available from Asota Mills, under product number CL10. Loop material 124 has an overall basis weight of about 44.1 g/m² (1.3 ounces per square yard (osy)) to about 54.2 g/m² (1.6 osy) (e.g., about 49.8 g/m² (1.47 osy)).

In order to form loop material 124, staple fibers 127 and 129 are provided on the surface of substrate 125 to form a staple fiber layer. The combination of substrate 125 and staple fibers 127 and 129 are then fed through a needling machine that includes forked needles positioned above the staple fibers 127, 129. As substrate 125 and staple fibers 127, 129 pass under the needles, the needles are driven through the staple fiber layer and substrate 125. Due to the construction of the needles, as the needles are driven downward through substrate 125, they carry some of staple fibers 127, 129 through substrate 125 to form loops 131. As the needles are then retracted out of substrate 125, the looped staple fibers 131 remain extending from the under side of substrate 125 opposite the staple fiber layer. Exemplary methods of forming loop materials similar to loop material 124 are described in greater detail in US 2004/0157036 and US 2009/0203280.

Referring again to Fig. 1, molten resin 126 is a blend of polypropylene and a small amount of polyethylene. The blend typically includes at least about 85 percent by weight (e.g., at least about 90 percent by weight, at least about 95 percent by weight) polypropylene and at most about 15 percent by weight (e.g., at most about 10 percent by weight, at most about 5 percent by weight) polyethylene. The small amount of polyethylene helps to enhance flow characteristics of molten resin 126 such that the molten resin can be delivered from extruder 114 to nip 108 in a highly controlled fashion.

Molten resin 126 is delivered to nip 108 between film 122 and loop material 124. Extruder 114 is positioned about 15.24 cm (six inches) to about 45.72 cm (18 inches) (e.g., about 30.48 cm (12 inches)) above nip 108. Molten resin 126 is extruded through extruder die 115 at a pressure of about 132.9 bar (2000 psi gauge) to about 206.8 bar (3000 psi gauge) (e.g., about 165.5 bar (2400 psi gauge)) and is delivered to the nip 108 at a temperature of about 260 degrees celsius (500 degrees Fahrenheit) to about 343.3 degrees celsius (650 degrees Fahrenheit) (e.g., about 304.4 degrees celsius (580 degrees Fahrenheit)). As a result, a layer of resin 126 that is about 0.0127 mm (0.5 mil) to about 0.0381 mm (1.5 mils) (e.g., about 0.0254 mm (one mil)) thick is formed between film 122 and loop material 124.

As shown in Fig. 1, extruder 114 has been adjusted such that molten resin 126 is delivered approximately into the center of nip 108. As a result, molten resin 126 contacts film 122 and loop material 124 at substantially the same time.

Rolls 102, 104, and 106 are configured to create a nip pressure of about 0.69 bar (10 psi gauge) to about 1.03 bar (15 psi gauge). In some embodiments, rolls 102, 104, and 106 are configured to created a nip pressure of about 0.83 bar (12 psi gauge); which equates to a pressure of about 5.44 kg (30 pounds) per linear 2.54 cm (inch). The pressure in pounds per linear 2.54 cm (inch) can be determined as a function of the gauge pressure and the diameter and length of pressure roll 102. The rotation of pressure roll 102 and backing roll 104 in opposite directions draws film 122, loop material 124, and molten resin 126 through nip 108. As these materials pass through nip 108, the pressures within nip 108 compress resin 126 between film 122 and loop material 124. The relatively low nip pressure helps to ensure that loops extending from loop material 124, which contact the peripheral surface of pressure roll 102, are not damaged (e.g., do not melt) during the lamination process.

In addition, pressure roll 102 and backing roll 104 are chilled to a temperature of about 10 degrees celsius (50 degrees Fahrenheit) to about 26.7 degrees celsius (80 degrees Fahrenheit) (e.g., about 18.3 degrees celsius (65 degrees Fahrenheit)). Cooled rolls 102 and 104 facilitate cooling of molten resin 126 as it passes through nip 108. In addition, the reduced temperature of rolls 102 and 104 help to prevent the loops of loop material 124 from melting due to the high temperature of molten resin 126.

It has also been found that the weight of substrate 125 of loop material 124 is sufficient to prevent loops 131 of loop material 124 from melting under the processing conditions discussed above. At the same time, loop material 124 allows the resulting composite material 121 to have a desired flexibility and, because loop material 124 is a relatively inexpensive material, helps to keep the cost of manufacturing composite material 121 well below the cost of manufacturing substantially air-impermeable methods using certain conventional manufacturing techniques.

Fig. 3 illustrates an enlarged schematic view of composite material 121. As shown in Fig. 3, composite material 121 includes a film layer formed of film 122, a loop material layer formed of loop material 124, and a resin layer formed of resin 126. These multiple layers are bonded together as a result of the lamination process described above. In particular, the portion of film 122 that contacts molten resin 126 during the lamination process melts and becomes welded to the resin layer. Similarly, as schematically illustrated in Fig. 3, the portions of substrate 125 and staple fibers 127, 129 of loop material 124 that contact molten resin 126 during the lamination process become encapsulated by molten resin 126 and melt such that loop material 124 becomes welded to the resin layer. Despite the high temperature of molten resin 126 and the fact that loop material 124 and molten resin 126 are formed primarily of the same type of material (i.e., polypropylene) and thus have similar melting temperatures, the hook-engageability of loops 131 of loop material 124 is preserved during the lamination process. The temperature and flow rate of molten resin 126, the temperature of chilled rolls 102, 104, and the construction of loop material 124 are selected to prevent loops 131 from melting and thus losing their hook-engageability. As a result, loops 131, which extend from the outer surface of the loop material layer of composite material 121, are hook-engageable. Composite material 121 can, for example, be engageable by the hooks of certain hook fastener products, such as HTH 819 hooks and/or HTH 851 hooks, both available from Velcro USA (Manchester, NH). Thus, as a result of the manufacturing process described above, composite material 121 can advantageously be used to make products that benefit from having one or more hook-engageable surfaces.

Referring again to Fig. 1, composite material 121 is embossed prior to being wound into a roll 123 around a winder 120. To emboss composite material 121, composite material 121 is passed through an embossing station 150, which includes an embossing roll 152 and a rubber (e.g., siliconized rubber) backing roll 154. A nip 156 is formed between embossing roll 152 and backing roll 154. Embossing roll 152 is a heated (e.g., steam-heated), steel roll that has a pattern of raised honeycomb-shaped areas 158 extending from its outer surface. Alternatively, embossing roll 152 can include any of various other raised patterns, such as combinations of diamonds, squares, triangles, circles, lines, curves (e.g., sinusoidal curves), logos, etc.

Embossing roll 152 is heated to a temperature of about 148.9 degrees celsius (300 degrees Fahrenheit) to about 204.4 degrees celsius (400 degrees Fahrenheit) (e.g., about 150.6 degrees celsius (303 degrees Fahrenheit)), and embossing roll 152 and backing roll 154 are configured to create a pressure of about 2.41 bar (35 psi gauge) to about 5.52 bar (80 psi gauge) (e.g., about 4.48 bar (65 psi gauge)) within nip 156. Composite material 121 is passed through nip 156 at a rate of about 6.1 m (20 feet) per minute to about 24.4 m (80 feet) per minute (e.g., about 9.1 m (30 feet) per minute). As composite material 121 passes through nip 156, raised areas 158 of embossing roll 152 contact the film layer of composite material 121 and compress adjacent regions of composite material 121 between embossing roll 152 and rubber backing roll 154, forming depressions in composite material 121. It is believed that these depressions act as natural hinge points that facilitate folding or bending of composite material 121, as will be discussed further below.

In certain embodiments, the loop material layer of composite material 121 provides composite material 121 with an average peel strength of about 30 grams per 2.54 cm (inch) width to about 100 grams per 2.54 cm (inch) width (e.g., about 35 grams per 2.54 cm (inch) width to about 45 grams per 2.54 cm (inch) width). A test developed by Velcro Group Corporation is used to determine the peel strength of composite material 121. The test involves the use of a 3.81 cm (1.5 inch) wide and 5.08 cm (2.0 inch) long sample of composite material 121 and a 2.54 cm (1.0 inch) wide by 2.54 cm (1.0 inch) long sample of 851 hook tape, manufactured by Velcro USA (Manchester, NH). A 2.54 cm (1.0 inch) wide by 5.08 cm (2.0 inch) wide piece of SMS nonwoven fabric is secured to the back of the hook sample using double-sided tape so that a 2.54 cm (1.0 inch) wide by 2.54 cm (1.0 inch) long tab of SMS nonwoven fabric extends from the end of the hook sample. The hook tape sample is superimposed upon the composite material sample (face to face) using minimal pressure (i.e., the amount of pressure necessary to create just enough hook and loop engagement so as to be able to continue with the test procedure). The hook tape and composite material samples are superimposed in a manner such that the machine direction of the hook sample is perpendicular to the machine direction of the loop material of the composite sample. A roll down machine having three rollers that each have a weight of about 2.04 kg (4.5 pounds), a width of about 4.45 cm (1.75 inches), and a durometer of 80 is then used to engage the loop material of the composite material sample with the hooks of the hook tape sample. The rollers of the roll down machine are rolled over the superimposed samples at a speed of 30.48 cm (12 inches) per minute. The rollers make two full cycles over the superimposed samples, where one full cycle constitutes rolling over the entire length of the superimposed samples and then back again. An example of a roll down machine that can be used for this procedure is the RD-3000 roll down machine available from ChemInstruments.

The SMS nonwoven fabric tab extending from the end of the hook sample and the corresponding end region of the composite material sample are then inserted into clamps of a tensile tester. The SMS nonwoven fabric tab extending from the hook tape sample is placed into a moveable, upper clamp of the tensile tester, and the end region of the composite material sample is placed into a stationary, lower clamp of the tensile tester. The clamps have a maximum gauge length (i.e., the opening between upper and lower clamps) of about 1.27 cm (0.5 inch). With the samples secured in the clamps, the upper clamp is moved away from the lower clamp at a rate of 30.48 cm (12 inches) per minute. The tensile tester is configured to begin recording data when a force of 0.02 kg (0.05 pounds) is measured, and the tensile tester is configured to move the jaws apart from one another until the jaws are separated by at least 3.81 cm (1.5 inches). An example of a suitable tensile tester is the MTS Sintech 1/S. The above-described test is performed at standard atmosphere ((21-25 degrees celsius 69.8-77.0 degrees Fahrenheit) and 45-55 percent relative humidity), and the test samples are preconditioned in standard atmosphere for 22-26 hours prior to testing.

As the jaws are separated from one another, the tensile tester measures the resistance force resulting from the engagement of the hooks and loops. This average measured force over the course of separation of the samples is then determined and divided by the width of the engaged samples (in this case, 2.54 cm (1.0 inch)) to determine the peel strength in grams per linear 2.54 cm (inch). The above-described test is repeated ten times and the average peel strength is determined based on those ten tests.

In some embodiments, the loop material layer of composite material 121 provides composite material 121 with an average shear strength of about 137.9 bar (2000 psi) to about 344.7 bar (5000 psi) (e.g., about 234.4 bar (3400 psi) to about 289.6 bar (4200 psi)). A test developed by Velcro Group Corporation is used to determine the shear strength of composite material 121. This test involves the use of a 3.89 cm (1.5 inch) wide by 5.08 cm (2.0 inch) long sample of composite material 121 and a 2.54 cm (1.0 inch) wide by 5.08 cm (2.0 inch) long sample of 851 hook tape, manufactured by Velcro USA (Manchester, NH). The hook tape sample is backed with a 2.54 cm (1.0 inch) wide by 5.08 cm (2.0 inch) long piece of masking tape to prevent breaking of the hook tape sample during the test. The hook tape sample is superimposed upon the composite material sample and the samples are engaged by a roll down machine in the manner described above. As a result, end regions of the samples overlap by 1.28 cm (0.5 inch). As a result, a 3.81 cm (1.5 inch) length of the composite material sample extends from one end of the overlapped region and is not facially engaged with the hook tape sample, and a 3.81 cm (1.5 inch) length of the hook tape sample extends from the opposite end of the overlapped region and is not facially engaged with the composite material sample. The hook tape and composite material samples are superimposed in a manner such that the machine direction of the hook sample and the machine direction of the loop material of the composite sample extend in the same direction. The free, non-facially engaged end regions of the hook tape and composite material samples are then inserted into clamps of a tensile tester (e.g., the MTS Sintech 1/S tensile tester). The end region of the hook tape sample is placed into a moveable, upper clamp of the tensile tester, and the end region of the composite material sample is placed into a stationary, lower clamp of the tensile tester. The clamps have a maximum gauge length (i.e., the opening between upper and lower clamps) of about 5.08 cm (2.0 inch). With the tabs of the samples secured in the clamps, the upper clamp is moved away from the lower clamp at a rate of 30.48 cm (12 inches) per minute. The clamps are moved apart a sufficient distance to achieve a shearing of the engaged hook tape and composite material samples. The above-described test is performed at standard atmosphere (21-25 degrees celsius (69.8-77.0 degrees Fahrenheit) and 45-55 percent relative humidity), and the test samples are preconditioned in standard atmosphere for 22-26 hours prior to testing.

As the jaws are separated from one another, the tensile tester measures the resistance force resulting from the engagement of the hooks and loops. The peak load (i.e., highest measured force) is then divided by the overlap area of the samples (in this case, 1.27 cm (0.5 inch) long by 2.54 cm (1.0 inch) wide) to determine the shear strength in kg per square cm (pounds per square inch). The above-described test is repeated ten times and the average shear strength is determined based on those ten tests.

Because each of the various components or layers of composite material 121 are formed primarily of the same type of material (i.e., polypropylene), composite material 121 is recyclable. As a result, composite material 121 can be readily used in many single-use or limited-use products, some of which are described below, in an environmentally conscious and cost-efficient manner.

In addition to being hook-engageable and recyclable, the combination of film 122, loop material 124, and the cooled resin 126 therebetween cooperate to make composite material 121 substantially air-impermeable. Composite material 121 can, for example, have an air-permeability of 0 cm³/s/cm² (0 ft³/min/ft²), as tested using ASTM D737-96.

After composite material 121 exits nip 108, the composite material is wound into a roll 123 around winder 120. Winder 120 can provide a tension of about 3.45 bar (50 psi gauge) to composite material 121 as composite material 121 is wound onto winder 120. Winding composite material 121 into a roll allows the composite material to be conveniently shipped or stored in mass quantities.

The exemplary method illustrated in Fig. 1 can be used to efficiently make composite material 121 at high rates of speed. For example, the method can be used to make composite material 121 at a rate of at least about 60.96 m (200 linear feet) per minute (e.g., at least 121.92 m (400 linear feet) per minute, at least about 182.88 m (600 linear feet) per minute). In some cases. composite material is produced at a rate of about 60.96 m (200 linear feet) per minute to about 243.84 (800 linear feet) per minute (e.g., about 121.92 m (400 linear feet) per minute to about 182.88 m (600 linear feet) per minute). The width of composite material 121 produced is typically about six feet wide. However, the composite material can be formed in wider or narrower swaths. As a result of the rapid production speeds of system 100, the exemplary method can be used to inexpensively manufacture material that is substantially air-impermeable, hook engageable, and recyclable.

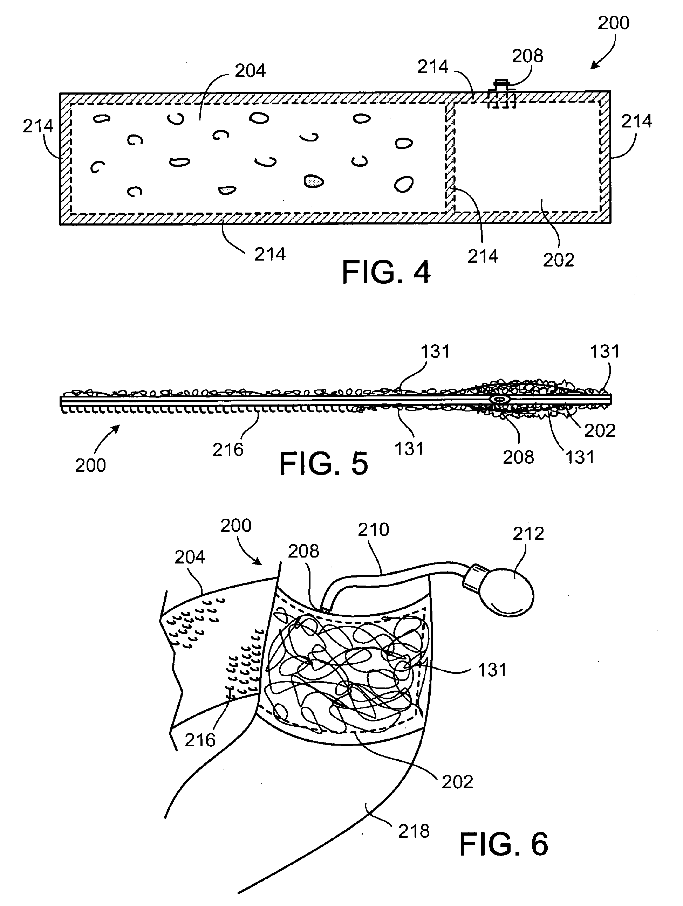

As discussed above, composite material 121 can be used to form any of various different products. An example of one such product is a medical compression device (e.g., a blood pressure cuff) 200, which is illustrated in Figs. 4 and 5. Blood pressure cuff 200 includes an inflatable pouch 202 and a strap portion 204 integrally extending from inflatable pouch 202. A fitment 208 capable of coupling with a flexible tube 210 (shown in Fig. 6) that is connected to a hand-operated pump 212 (also shown in Fig. 6) is in fluid communication with inflatable pouch 202. As described below, inflatable pouch 202 and strap portion 204 can be formed by folding a sheet of composite material 121 and then welding the overlapping halves of the folded sheet of composite material 121 together. As a result of this process, welds 214 are formed around the perimeter of inflatable pouch 202 and around the perimeters of strap portion 204.

Referring to Fig. 5, loops 131 of the loop material layer of composite material 121 extend from the outer surfaces of inflatable pouch 202 and strap portion 204. Loops 131, as discussed above, are hook-engageable. In addition, loops 131 provide a soft, comfortable surface for contacting the user's skin during use. In addition, a hook fastener 216 including an array of loop-engageable hooks is attached (e.g., welded) to an outer surface of strap portion 204. The hook fastener 216 is arranged so that when blood pressure cuff 200 is wrapped around a patient's arm and strap portion 204 overlaps itself, hook fastener 216 can releasably engage loops 131 extending from the opposite outer surface of strap portion 204 to tightly secure blood pressure cuff 200 around the patient's arm. The loop-engageable hooks of hook fastener 216 can, for example, be of molded form available from Velcro, USA under designation HTH 819, HTH 851, or any of various other hooks that are capable of releasably engaging loops 131 of composite material 121. Typically, the hooks are formed primarily of (e.g., entirely of) the same polymer as the various components of composite material 121. In certain embodiments, for example, hook fastener 216, film 122 of composite material 121, and loop material 124 of composite material 121 are formed substantially entirely of polypropylene, and resin 126 of composite material 121 is formed primarily of polypropylene. As discussed above, for example, the resin layer of composite material 121 can include at least about 85 percent by weight (e.g., at least about 90 percent by weight, at least about 95 percent by weight) polypropylene and at most about 15 percent by weight (e.g., at most about 10 percent by weight, at most about 5 percent by weight) polyethylene.

Fig. 6 shows blood pressure cuff 200 wrapped around the upper portion of a patient's arm 218. Flexible tube 210, mated with fitment 208, is used as a conduit extending from pump 212 to fill inflatable pouch 202 with air as pump 212 is operated. This tightens blood pressure cuff 200 around the patient's arm 218 until blood flow is constricted, and enables measurement of blood pressure during gradual release of air from pouch 202. Typically, automatic blood pressure detecting devices with which blood pressure cuff 200 can be used are adapted to sense (e.g., by audible signal) blood passing through the patient's arm both as blood pressure cuff 200 is inflated and as blood pressure cuff 200 is deflated. By sensing blood passing through the patient's arm as the cuff is being inflated, the blood pressure detecting device is able to determine the point at which blood can no longer be detected flowing through the patient's arm. At this point the cuff is slowly deflated. The points at which blood flow is first sensed and last sensed by the blood pressure detecting device as the cuff is being deflated can be used to indicate the systolic and diastolic blood pressure of the patient.

It has been found that the embossed pattern of composite material 121 helps to reduce the noise associated with inflating and deflating blood pressure cuff 200. Without wishing to be bound by theory, it is believed that the depressions formed in composite material 121 during the embossing process act as hinges and thus decrease bending resistance of composite material 121. It is further believed that this decreased bending resistance decreases the noise associated with inflating and deflating blood pressure cuff 200, and thus improves the ability of the blood pressure detecting device to accurately identify the point at which blood flow through the patient's arm is no longer audible. This can, for example, decrease the likelihood of the blood pressure detecting device being prematurely unable to sense blood flow through the patient's arm as a result of background noise caused by the inflating cuff. As a result, the likelihood of the blood pressure detection device deflating blood pressure cuff 200 prematurely and having to restart the blood pressure detection procedure can be reduced.

Due to the efficiency with which composite material 121 can be manufactured using the various methods described herein, blood pressure cuff 200 can be manufactured relatively inexpensively. This allows blood pressure cuff 200 to be manufactured as a limited use (e.g., single-use) device that can, for example, be used in trauma rooms or other places where the cuff may become contaminated with blood or other bodily fluids during use. After becoming contaminated, the blood pressure cuff 200 can simply be discarded. In addition, because the various components of composite material 121 are formed primarily of the same material (e.g., polypropylene), blood pressure cuff 200 is readily recyclable. This is a beneficial characteristic for products such as blood pressure cuff 200, which will be used only a limited number of times (e.g., ten times or fewer, or only once) before being discarded.

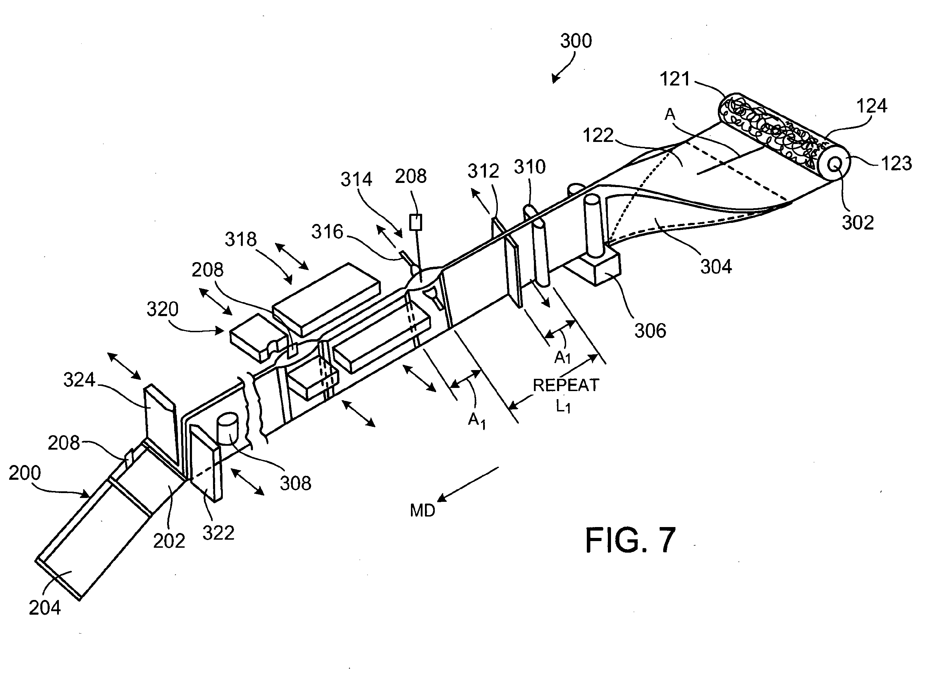

Fig. 7 schematically illustrates a method of manufacturing multiple medical compression devices (e.g., blood pressure cuffs) of the type illustrated in Figs. 4-6. As shown in Fig. 7, this exemplary method uses a horizontal poucher system 300 to overlap (e.g., by folding) and weld a single sheet of composite material 121 in selected bonding regions to form inflatable pouch 202 and strap portion 204 of blood pressure cuffs 200. Beginning at the right side of Fig. 7, the sheet of composite material 121 is led from composite roll 123, which is positioned on a rotatable shaft 302, into a former 304 where the composite material 121 is folded about an axis A to form overlapped areas of composite material 121. Once centered on former 304, composite material 121 passes through drive stations 306 and 308, which are located near the entry and exit, respectively, of system 300 and are coupled to act in unison. Drive station 306 includes rolls extending the full width of the folded composite material to pull the sheet-form composite through the former 304. In coordination with the downstream drive station 308, drive station 306 also tensions the material. Due to the arrangement of composite material 121 on composite roll 123, after being folded, folded regions of film 122 contact one another and loops 131 extend from the outer surfaces of the folded sheet. As a result, the outer surfaces of the folded composite material 121 are hook-engageable.

By indexing action, the sheet form composite material 121 passes from former 304 to a pair of welding stations 310 and 312 where sealing bars weld selected overlapped regions of the folded composite material 121 together. The folded composite material 121 then passes from the weld stations 310 and 312 to an insertion station 314. As the folded composite material 121 passes through insertion station 314, portions of the folded composite material 121 between welds formed by weld stations 310 and 312 are engaged by a pair of oppositely acting suction cups 316. The top of the pouch between the welds is opened to enable the placement of fitment 208 in an opening between the welds.

One alternative method for opening the sides of the pouch is to use loop-engageable fasteners (e.g., hook fasteners) to engage and pull back the loop bearing sides of the folded composite material 121. This can be used to open the sides slightly, at which time spreader blades can be inserted and spread apart to complete the action. Another method for opening is to place the fold axis A slightly off center of the overall web width. Thus, when folded about axis A, one edge of the folded material will extend higher than the other. Given height difference between opposed edges, high-pressure air blown into the pouch area or mechanical means such as pinchers can open the sides.

With fitment 208 held in place at the opening, downstream drive station 308, in conjunction with drive station 306, indexes the folded and welded material from insertion station 314 to a top seal station 318. Sealing jaws of top seal station 318 with motion similar to that of the heat seal bars at weld stations 310 and 312, can be moved inward to weld the top edge of the folded composite material 121 and can be moved outwardly to release the top edge of the folded composite material 121. While system 300 is forming the welds at weld stations 310 and 312, and inserting fitment 208 at insertion station 314, the sealing jaws at top seal station 318 engage to seal shut the top of the folded composite material 121 along the portion of the folded composite material 121 that ultimately becomes strap portion 204 of blood pressure cuff 200. Simultaneously, sealing jaws at an adjacent top seal station 320 engage to weld the top edge of the folded composite material 121 to form inflatable pouch 202 of blood pressure cuff 200. This weld also functions to secure fitment 208 in place. As discussed above, fitment 208 can subsequently be connected to conduit 210 and pump 212 to allow inflation of pouch 202.

Downstream cutting jaws 322, 324 sever the folded composite material 121 along the trailing weld adjacent inflatable pouch 202. This severs the leading blood pressure cuff preform from the remainder of the continuous sheet form composite material 121 that is in the process of being formed into additional blood pressure cuff preforms. After severing the leading blood pressure cuff preform from the remainder of composite material 121, hook fastener 216 is welded to the blood pressure cuff preform to complete the formation of blood pressure cuff 200.

A repeat length L₁ for system 300 is established by the desired length of blood pressure cuff 200 and extends from sealing bar 310 to the downstream weld that was previously formed by sealing bar 310. Similarly, the distance A1 between weld stations 310 and 312 are established by the desired length of inflatable pouch 202 of blood pressure cuff 200.

While certain embodiments have been described, other embodiments are possible.

In certain embodiments, for example, the embossing of composite material 121 can be performed by an embossing station that is separate from system 100. In such embodiments, for example, composite material 121 can be wound onto roll and then transported to an embossing station, where the roll of composite material 121 is unwound and passed through a nip formed between an embossing roll and backing roll of the type described above.

While composite material 121 has been described as being embossed, in certain embodiments, composite material 121 is not embossed prior to being formed into a product, such as a compression device. In some embodiments, as an alternative to or in addition to embossing composite material 121, a patterned film can be introduced into nip along with molten resin and loop material. The patterned film can facilitate folding of the material and, in the case of blood pressure cuffs formed from the composite material, can help to reduce noise created by the cuff as it is inflated.

While support roll 106 of system 100 has been described as contacting pressure roll 102 to prevent or reduce bowing of pressure roll 102 as a result of high pressures within nip 108, in certain embodiments, support roll 106 is moved to a disengaged position such that support roll 106 does not contact pressure roll 102. Such an arrangement can be used, for example, when pressures within nip 108 are not sufficiently high to cause bowing of pressure roll 102. As an alternative to moving support roll 106 to a disengaged position, support roll 106 can be entirely removed from system 100 in certain cases.

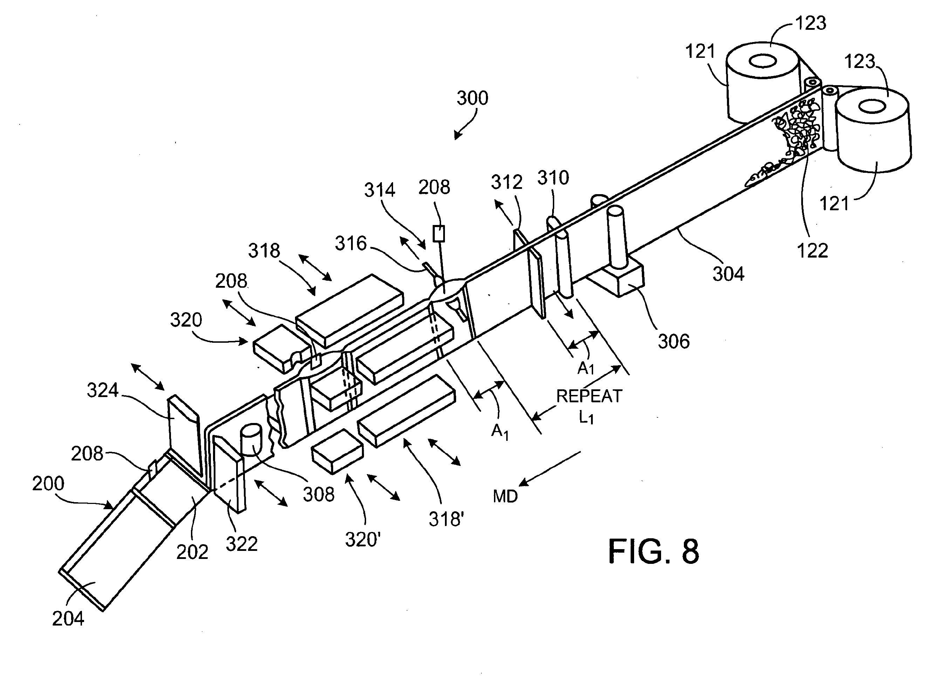

While the compression devices (e.g., blood pressure cuffs) 200 have been described as being manufactured by folding composite material 121 and then welding overlapping portions of the composite material together, other types of manufacturing techniques can be used. In certain embodiments, as schematically illustrated in Fig. 8, for example, two separate sheets of composite material 121 are welded together to form the compression devices. The two sheets of composite material 121 are fed from rolls 123 in an overlapping manner. The sheets are welded together using a welding process similar to the welding process described above with respect to Fig. 7. However, in addition to using top seal stations 318 and 320 to provide welds, bottom seal stations 318' and 320' are provided to weld the overlapped sheets of composite material 121 along the bottom edge of those sheets. The other steps of the manufacturing process can be substantially the same as those steps described above with respect to Fig. 7.

While the above-described manufacturing methods describe welding the hook fasteners to composite material 121 after the composite material or materials has/have been formed into the shape of the blood pressure cuffs, the hook fasteners can alternatively be welded onto composite material 121 prior to forming the composite material into the shape of blood pressure cuffs. For example, before winding composite material 121 onto winder 120, composite material can pass through a welding station that welds hook fasteners at longitudinally spaced apart regions of the composite material. As a result, after forming the composite material into blood pressure cuffs, the hook fasteners will be located only along a desired portion of the length of the blood pressure cuffs.

Alternatively, prior to winding composite material 121 onto winder 120, the composite material can pass through a welding station that continuously welds one or more hook fastener strips to the composite material. The hook fastener strips can, for example, be provided in a roll and can be delivered to a nip formed between a support roll and a thermal sealing member. In some embodiments, the hook fastener strips can be provided as continuous strips that extend along the entire length of the blood pressure cuff.

While the hook fasteners have been described as being welded to composite material 121, other techniques can be used. In some embodiments, for example, the hook fasteners are joined in situ to composite material 121. In such embodiments, composite material 121 may be passed through a nip formed between a mold roll including hook-shaped cavities and a pressure roll along with molten resin. The molten resin is pressed into the hook-shaped cavities to form hooks. At the same time, the molten resin bonds to composite material 121. The general concept of in situ lamination is explained in U.S. 5,260,015 by Kennedy et al., and in situ lamination of strips of molded hooks, per se, is disclosed in U.S. 6,205,623 by Shepard et al..

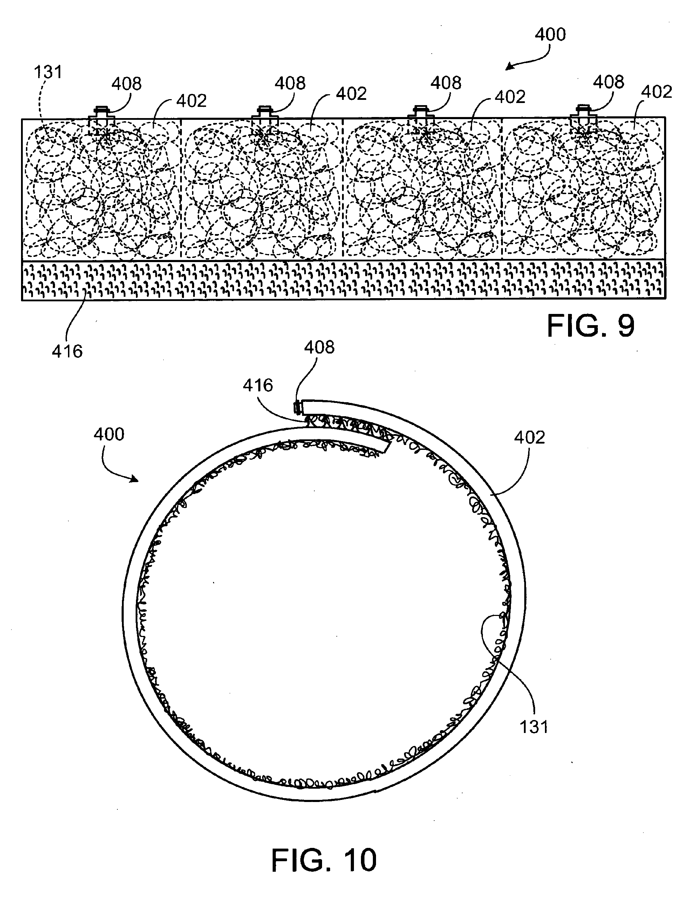

While the compression devices 200 have been described as blood pressure cuffs, other types of compression devices can be manufactured using methods similar to those described above. Fig. 9, for example, illustrates a sequential compression device 400 that can be manufactured using the composite material described above and using methods similar to those described above. Sequential compression device 400 is a disposable wrap that includes multiple inflatable compartments 402. A fitment 408 is connected to each of the inflatable compartments 402. Fitments 408 permit a tube from a pump (e.g., an air pump) to be connected thereto to allow inflatable compartments 402 to be selectively inflated and deflated. A hook fastener strip 416 is attached to the polymeric film of the composite material of compression device 400 along the lower edge of compression device 400. Loop material 131 extends from the outer surface of the composite material opposite hook fastener strip 416, and thus is shown in dashed lines. As shown in Fig. 10, hook fastener strip 416 can engage loop material 131 to form a sleeve having multiple annular inflatable compartments along its length.

During use, sequential compression device 400 is secured in the form of a sleeve around a limb (e.g., a leg) of a patient by wrapping the composite material around the limb of the patient and then fastening the hooks of hook fastener strip 416 to loop material 131. Each of the inflatable compartments 402 is then connected to a pump (e.g., an air pump) via separate tubes. Compartments 402 are then sequentially inflated and deflated. Using sequential compression device 400 in this manner can improve blood flow and prevent clot formation. Such use can help to prevent the development of deep vein thrombosis (DVT) and similar conditions in immobile patients.

Sequential compression devices 400 can be formed using systems and methods similar to those illustrated in Figs. 7 and 8. However, the system would typically be equipped with additional welding stations that can be used to form the increased number of inflatable compartments 402 along the length of sequential compression device 400. In addition, hook fastener strip 416 would be applied along one of the lengthwise edges of compression device 400.

While compression device 400 has been described as having a hook fastener strip that engages loop material to form a sleeve, in certain embodiments, opposite edge regions of the compression device are thermally welded to form a permanent sleeve. In such embodiments, prior to use, the compression device would be slid onto the limb of the patient rather than being wrapped around the patient's limb and then fastened.

In addition to blood pressure cuffs and sequential compression devices, various other types of compression devices can be manufactured using the composite material described above. One such device is an inflatable tourniquet cuff. The inflatable tourniquet cuff can be formed and operated in substantially the same manner as the blood pressure cuffs described above. Tourniquet cuffs can, however, be provided in a wide range of sizes to allow the tourniquets be used on various different body parts of different patients. Some inflatable tourniquet cuffs can, for example, be sized to fit around a patient's arm and other inflatable tourniquet cuffs can be sized to fit around a patient's leg. Such tourniquet cuffs can be used to prevent blood flow to the patient's arm or leg during use. For example, during a surgical procedure on a patient's arm, the arm-sized cuff can be wrapped around the patient's arm and then inflated to stop blood flow to the patient's arm. Similarly, during a surgical procedure on a patient's leg, the arm-sized cuff can be wrapped around the patient's leg and then inflated (e.g., by connecting a tube from a pneumatic pump to the fitment of the tourniquet cuff and operating the pump) to stop blood flow to the patient's arm. Use of the tourniquet cuffs in this manner can permit the surgeon to work in a bloodless operative field. Smaller tourniquet cuffs can also be made for use on smaller limbs, such as fingers and toes, of patients.

Another type of device that can be manufactured using the composite materials described above is an inflatable immobilizing device, such as an inflatable cast. Such casts can, for example, be sized and shaped for use on a patient's ankle or wrist.

While film 122 has been described as a two mil thick, cast, polypropylene film. Other types of films can alternatively be used. For example, films having a thickness of about 0,025 mm (1 mil) to about 0,127 mm (5 mils) can be used.

While loop material 124 has been described as including a 17 grams per square meter spunbond polypropylene substrate through which staple fibers 127 and 129 are needled, other types of nonwoven fabrics can alternatively be used to form the substrate of the loop material. In some embodiments, for example, staple fibers 127 and 129 are needled through a 30 grams per square meter polypropylene SMS substrate to form the loop material. In such embodiments, any of the various needling methods described or referenced above can be used to make the loop material. The resulting loop material has an overall basis weight of about 0.058 kg/m² (1.7 osy) to about 0.068 kg/m² (2.0 osy).

In some embodiments, materials other than nonwoven materials are used as the substrate of the needled loop material. Examples of such materials include films and knits.

While loop material 124 has been described as including loops formed of staple fibers available from Asota Mills, under product numbers L10D and CL10, it should be understood that various other types of staple fibers can be used so long as those staple fibers are capable of being needled through a substrate to form loops and are formed of a material desired for the particular composite material being formed (e.g., formed substantially entirely of a certain type of polymeric material, such as polypropylene).

As an alternative to the needled loop materials described above, in certain embodiments, other types of hook-engageable materials can be used. Examples of such hook-engageable materials include SMS fabric, spunbond fabric (e.g., spunbond polypropylene (commodity grade)), and knits. However, we have found the above-described needled loop materials to be particularly advantageous in certain cases because they typically provide longer useful life expectancies, and it is believed that the needled loop materials are particularly effective at preventing the hook-engageable fibers (i.e., loops) from being destroyed (e.g., by melting) during manufacturing processes that involve the application of molten resin to the needled loop material.

While molten resin 126 has been described as a mixture of polymers (i.e., polypropylene and polyethylene), in certain embodiments, the molten resin is formed entirely of a single polymer (e.g., polypropylene).

While the film, hook-engageable material, and molten resin of those composite materials discussed above have been described as being formed primarily of or entirely of polypropylene, they can alternatively be formed primarily of or entirely of other materials. In certain embodiments, for example, the film, hook-engageable material, and molten resin are formed primarily of or entirely of polyethylene. Composite materials of this construction can, for example, be advantageous for applications that benefit from increased flexibility or stretchability while still providing a substantially air-impermeable barrier. Alternatively, the film, hook-engageable material, and molten resin can be formed primarily of or entirely of nylon (e.g., lightweight nylon knit).

While certain composite materials discussed above have been described as being substantially air-impermeable, in certain embodiments, the composite material is not air-permeable. In some embodiments, for example, the composite material is liquid-impermeable but is air-permeable. One example of such a composite material is made with a perforated polymeric film. The perforations are sized such that liquid is unable to pass through the film while air and other gases are allowed to pass through the perforations. Such composite materials can be useful, for example, for forming products that benefit from liquid-impermeability but are not required to be inflatable.

In addition to the compression devices described above, any of various other types of products can be manufactured using the composite materials described herein. In some embodiments, for example, medical cold wraps can be formed using manufacturing processes similar to those described above. In certain embodiments, for example, rather than placing a valve or fitment into a pouch formed by the composite material or materials, the pouch can be filled with a cooling liquid, such as a cooling chemical solution or water, and then the composite material or materials can be welded in a manner to seal the cooling liquid within the pouch. Such a product can, for example, be used as a medical cold wrap that can be secured around a body part of a user to cool the body part. To use this type of product, the product would be placed in a freezer until the cooling liquid is cooled to a desired temperature and then wrapped around the user's body part and fastened to cool the body part. Such products are useful, for example, to reduce swelling of the body part. Examples of similar wraps are described in US 2004/0181156. In addition to the above-described compression devices and medical wraps, any of the various other types of products described in US 2004/0181156 can be manufactured using the composite materials described herein.

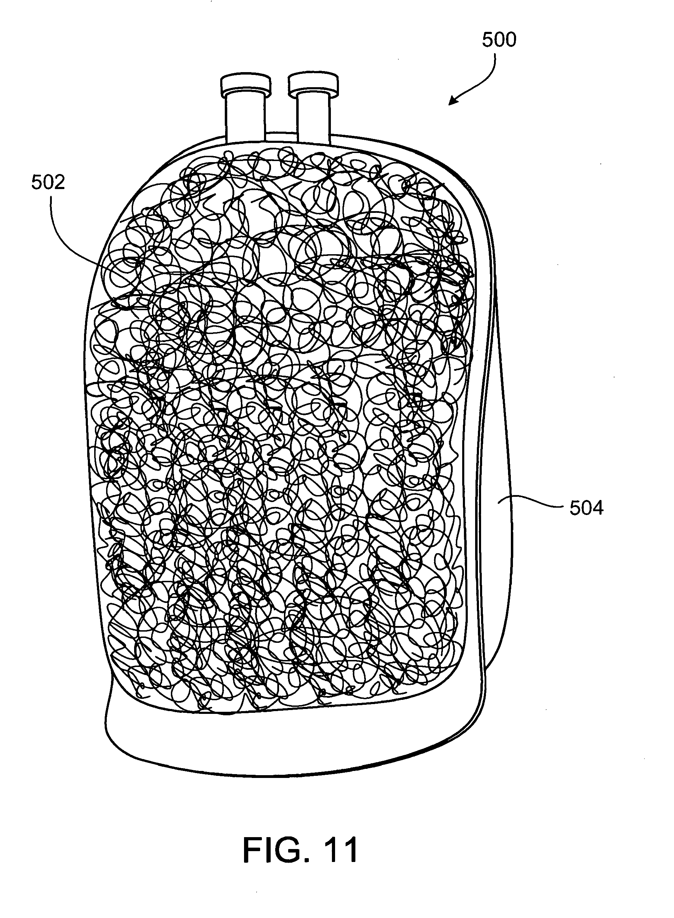

In some embodiments, the composite material is used in the construction of urinary drain bags. As shown in Fig. 11, for example, a urinary drain bag 500 includes a first wall 502 formed from the liquid-impermeable and hook-engageable composite material of the type described above. A second wall 504 of drain bag 500 is formed of a liquid-impermeable polymeric film. The loop layer of the composite material is exposed along an outer surface of the rear wall of drain bag 500. In this manner, drain bag 500 can be releasably fixed to any surface having engageable hooks extending therefrom. In certain cases, for example, hook tape can be applied to a hospital bed and drain bag 500 can be conveniently secured to the hospital bed by engaging the loops of the composite material to the hooks of the hook tape. Such an arrangement can make it easier for drain bags to be replaced by medical personnel. Any of various other types of medical bags that would benefit from being releasably secured to a surface can similarly be formed from the hook-engageable composite materials described herein.

Referring again to Fig. 1, while molten resin 126 has been described as being introduced directly into nip 108 such that molten resin 126 contacts film 122 and loop material 124 at substantially the same time, in certain embodiments, molten resin 126 is applied first to film 122, and film 122 then carries molten resin 126 into nip 108. This technique can provide molten resin 126 with additional time to cool prior to contacting loop material 124, which can help to prevent loops 131 extending from the outer surface of loop material 124 from melting and becoming bonded together. As a result, this technique can help to ensure that loops 131 extending from loop material 124 remain capable of engaging hooks of hook fastener elements after loop material 124 is bonded to film 122 to form the composite material.

While the composite materials of embodiments above have been described as including a hook-engageable surface, in certain embodiments, composites having only surfaces that are not hook-engageable are formed. Such composites can be formed using manufacturing processes similar to those described above. In certain embodiments, for example, a needled loop material having a 17 grams per square meter spunbond polypropylene substrate is fed into nip 108 formed between pressure roll 102 and backing roll 104. Molten resin is applied to the loop material to form a resin layer that is about 0,051 mm (2 mils) thick. The loop material then carries the molten resin into nip 108. Due to the relatively thin substrate of the loop material, the increased amount of molten resin, and the extended duration of contact between the molten resin and the loop material, the molten resin drowns the loops extending from the loop material. This causes the loops extending from the outer surface of the loop material (i.e., the surface in contact with the PTFE coated roll) to melt and become bonded to one another. As a result, the loop material layer of the resulting composite material does not readily engage hooks of hook fastener elements. Such a construction can, for example, be advantageous for products in which a soft non-hook-engageable outer surface is desired.

Other embodiments are within the scope of the following claims.

## Claims

1. A method of forming a composite material, the method comprising:
introducing a sheet-form film (122) and a hook-engageable material (124) into a nip (108) formed between a first roll (102) and a second roll (104);
introducing molten resin (126) into the nip (108) between the film (122) and the hook-engageable material (124); and
allowing the molten resin (126) to cool so that the film (122) becomes attached to the hook-engageable material (124) by the cooled resin,
wherein each of the film (122), the hook-engageable material (124), and
the molten resin (126) includes at least about 85 percent by weight of a first polymer.

2. The method of claim 1, wherein the composite material is substantially air-impermeable.

3. The method of claim 1 or 2, wherein the composite material has an air-permeability of 0 cm³/s/cm².

4. The method of any of the above claims, wherein fibers (127, 129) extending from a surface of the hook-engageable material are capable of engaging hooks of hook fasteners after the film is attached to the hook-engageable material by the cooled resin.

5. The method of any of the above claims, wherein the first polymer is polypropylene.

6. The method of any of the above claims, wherein the film (122) and the hook-engageable material (124) consist essentially of polypropylene.

7. The method of any of the above claims, wherein the molten resin (126) is at least about 90 percent by weight polypropylene.

8. The method of any of the above claims, wherein the molten resin (126) further comprises polyethylene.

9. The method of any of the above claims, wherein the hook-engageable material (124) comprises a 17 grams per square meter spunbond polypropylene substrate through which a plurality of loop shaped staple fibers extend.

10. The method of any of claims 1-8, wherein the hook-engageable material (124) comprises a 30 grams per square meter SMS polypropylene substrate through which a plurality of loop shaped staple fibers extend.

11. The method of any of the above claims, wherein the molten resin (126) introduced into the nip (108) has a thickness of about 0.0254 mm [one mil], the film (122) has a thickness of about 0.0762 mm [three mils], and the hook-engageable material (124) has a weight of about 44.1 g/m² to about 67.8 g/m² [about 1.3 to about 2.0 osy].

12. The method of any of the above claims, wherein the hook-engageable material (124) has a basis weight of about 44.1 g/m² to about 54.2 g/m² [about 1.3 to about 1.6 osy].

13. The method of any of the above claims, wherein the molten resin (26) is at a temperature of at least about 260 degrees Celsius [about 500 degrees Fahrenheit] when introduced into the nip (108).

14. The method of any of the above claims, wherein the molten resin (126) is at a temperature of about 287.8 degrees Celsius to about 315.6 degrees Celsius [about 550 degrees Fahrenheit to about 600 degrees Fahrenheit] when introduced into the nip (108).

15. The method of any of the above claims, wherein the molten resin (126) is at a temperature of about 304.4 degrees Celsius [about 580 degrees Fahrenheit] when introduced into the nip (108).

16. The method of any of the above claims, wherein the film (122) is a cast film.

17. The method of any of the above claims, wherein the molten resin (126) is introduced into the nip (108) in a manner such that the molten resin (126) contacts the film (122) and the hook-engageable (124) material at substantially the same time.

18. The method of any of the above claims, wherein the molten resin (126) is introduced into the nip (108) by applying the molten resin (126) to the hook-engageable material (124) and rotating the first and second rolls (102, 104) to carry the film (122), the hook-engageable material (124), and the molten resin (126) into the nip.

19. The method of any of claims 1-17, wherein the molten resin (126) is introduced into the nip (108) by applying the molten resin (126) to the film (122) and rotating the first and second rolls (102, 104) to carry the film (122), the hook-engageable material (124), and the molten resin (126) into the nip (108).

20. The method of any of the above claims, wherein at least one of the first and second rolls (102, 104) is chilled to facilitate cooling of the molten resin (126).

21. The method of any of the above claims, wherein both of the first and second rolls (102, 104) are chilled.

22. The method of any of the above claims, further comprising embossing the composite material.

23. The method of any of the above claims, wherein embossing the composite material comprises passing the composite material between an embossing roll (152) and a backing roll (154), the embossing roll (152) have a plurality of raised features (158) that compress the composite material between the embossing roll (152) and the backing roll (154).

24. The method of claim 23, wherein the embossing roll (152) is a heated roll.

## Patentansprüche

1. Verfahren zum Bilden eines Verbundmaterials, wobei das Verfahren umfasst:
Einführen einer blattförmigen Folie (122) und eines von Haken eingreifbaren Materials (124) in einen Walzenspalt (108), der zwischen einer ersten Walze (102) und einer zweiten Walze (104) gebildet ist;
Einführen von geschmolzenem Harz (126) in den Walzenspalt (108) zwischen die Folie (122) und das von Haken eingreifbare Material (124);
und
Zulassen, dass das geschmolzene Harz (126) abkühlt, so dass die Folie (122) an dem von Haken eingreifbaren Material (124) befestigt wird durch das abgekühlte Harz,
wobei die Folie (122), das von Haken eingreifbare Material (124) und das geschmolzene Harz (126) jeweils mindestens ungefähr 85 Gewichtsprozent eines ersten Polymers beinhalten.

2. Verfahren nach Anspruch 1, wobei das Verbundmaterial im Wesentlichen luftundurchlässig ist.

3. Verfahren nach Anspruch 1 oder 2, wobei das Verbundmaterial eine Luftdurchlässigkeit von 0 cm³/s/cm² hat.

4. Verfahren nach irgendeinem der obigen Ansprüche, wobei Fasern (127, 129), die sich von einer Oberfläche des von Haken eingreifbaren Materials erstrecken, geeignet sind, um in Haken von Hakenbefestigern einzugreifen nachdem die Folie an dem von Haken eingreifbaren Material über das abgekühlte Harz befestigt ist.

5. Verfahren nach irgendeinem der obigen Ansprüche, wobei das erste Polymer Polypropylen ist.

6. Verfahren nach irgendeinem der obigen Ansprüche, wobei die Folie (122) und das von Haken eingreifbare Material (124) im Wesentlichen aus Polypropylen bestehen.

7. Verfahren nach irgendeinem der obigen Ansprüche, wobei das geschmolzene Harz (126) mindestens 90 Gewichtsprozent Polypropylen hat.

8. Verfahren nach irgendeinem der obigen Ansprüche, wobei das geschmolzene Harz (126) außerdem Polyethylen umfasst.

9. Verfahren nach irgendeinem der obigen Ansprüche, wobei das von Haken eingreifbare Material (124) ein 17 Gramm pro Quadratmeter schweres Spinnpolypropylen-Substrat umfasst, durch das sich eine Vielzahl an schleifenförmigen Stapelfasern erstreckt.

10. Verfahren nach irgendeinem der Ansprüche 1-8, wobei das von Haken eingreifbare Material (124) ein 30 Gramm pro Quadratmeter schweres SMS Polypropylen-Substrat umfasst, durch dass sich eine Vielzahl an schleifenförmigen Stapelfasern erstreckt.

11. Verfahren nach irgendeinem der obigen Ansprüche, wobei das geschmolzene Harz (126), das in den Walzenspalt (108) eingeführt wird eine Dicke von ungefähr 0,0254 mm [ein mil] hat, die Folie eine Dicke von ungefähr 0,0762 mm [drei mils] hat und das von Haken eingreifbare Material (124) ein Gewicht von ungefähr 44,1 g/m² bis ungefähr 67,8 g/m² [ungefähr 1,3 bis ungefähr 2,0 osy] hat.

12. Verfahren nach irgendeinem der obigen Ansprüche, wobei das von Haken eingreifbare Material (124) ein Basisgewicht von ungefähr 44,1 g/m² bis ungefähr 54,2 g/m² [ungefähr 1,3 bis ungefähr 1,6 osy] hat.

13. Verfahren nach irgendeinem der obigen Ansprüche, wobei das geschmolzene Harz (126) eine Temperatur von mindestens ungefähr 260 Grad Celsius [ungefähr 500 Grad Fahrenheit] hat, wenn es in den Walzenspalt (108) eingeführt wird.

14. Verfahren nach irgendeinem der obigen Ansprüche, wobei das geschmolzene Harz (126) eine Temperatur von ungefähr 287,8 Grad Celsius bis ungefähr 315,6 Grad Celsius [ungefähr 550 Grad Fahrenheit bis ungefähr 600 Grad Fahrenheit] hat, wenn es in den Walzenspalt (108) eingeführt wird.

15. Verfahren nach irgendeinem der obigen Ansprüche, wobei das geschmolzene Harz (126) eine Temperatur von ungefähr 304,4 Grad Celsius [ungefähr 580 Grad Fahrenheit] hat, wenn es in den Walzenspalt (108) eingeführt wird.

16. Verfahren nach irgendeinem der obigen Ansprüche, wobei die Folie (122) eine gegossene Folie ist.

17. Verfahren nach irgendeinem der obigen Ansprüche, wobei das geschmolzene Harz (126) in den Walzenspalt (108) in einer Weise eingeführt wird, so dass das geschmolzene Harz (126) die Folie (122) und das von Haken eingreifbare Material (124) im Wesentlichen gleichzeitig berührt.

18. Verfahren nach irgendeinem der obigen Ansprüche, wobei das geschmolzene Harz (126) in den Walzenspalt (108) eingeführt wird indem das geschmolzene Harz (126) auf das von Haken eingreifbare Material (124) aufgetragen wird und die ersten und zweiten Walzen (102, 104) gedreht werden, um die Folie (122), das von Haken eingreifbare Material (124) und das geschmolzene Harz (126) in den Walzenspalt (108) zu befördern.

19. Verfahren nach irgendeinem der Ansprüche 1-17, wobei das geschmolzene Harz (126) in den Walzenspalt (108) eingeführt wird indem das geschmolzene Harz (126) auf die Folie (122) aufgetragen wird und die ersten und zweiten Walzen (102, 104) gedreht werden, um die Folie (122), das von Haken eingreifbare Material (124) und das geschmolzene Harz (126) in den Walzenspalt (108) zu befördern.

20. Verfahren nach irgendeinem der obigen Ansprüche, wobei mindestens eine der ersten und zweiten Rollen (102, 104) gekühlt wird, um das Abkühlen des geschmolzenen Harzes (126) zu unterstützen.

21. Verfahren nach irgendeinem der obigen Ansprüche, wobei beide der ersten und zweiten Rollen (102, 104) gekühlt werden.

22. Verfahren nach irgendeinem der obigen Ansprüche, außerdem umfassend Prägen des Verbundmaterials.

23. Verfahren nach irgendeinem der obigen Ansprüche, wobei Prägen des Verbundmaterials Durchleiten des Verbundmaterials zwischen einer Prägewalze (152) und einer Gegenwalze (154) umfasst, wobei die Prägewalze (152) eine Vielzahl an erhabenen Merkmalen (158) hat, die das Verbundmaterial zwischen die Prägerolle (152) und die Gegenrolle (154) presst.

24. Verfahren nach Anspruch 23, wobei die Prägerolle (152) eine beheizte Rolle ist.

## Revendications

1. Procédé de formation d'un matériau composite, le procédé comportant les étapes qui consistent à :
introduire un film (122) en forme de feuille et un matériau (124) apte à être engagé par crochets dans un interstice (108) formé entre un premier cylindre (102) et un deuxième cylindre (104),
introduire de la résine fondue (126) dans l'interstice (108), entre le film (122) et le matériau (124) apte à être engagé par crochets et
laisser refroidir la résine fondue (126) de telle sorte que le film (122) soit fixé par la résine refroidie sur le matériau (124) apte à être engagé par crochets,
le film (122), le matériau (124) apte à être engagé par crochets et la résine fondue (126) comprenant au moins environ 85 pour cent en poids d'un premier polymère.

2. Procédé selon la revendication 1, dans lequel le matériau composite est essentiellement imperméable à l'air.

3. Procédé selon les revendications 1 ou 2, dans lequel le matériau composite présente une perméabilité à l'air de 0 cm³/s/cm².

4. Procédé selon l'une quelconque des revendications précédentes, dans lequel des fibres (127, 129) débordant d'une surface du matériau apte à être engagé par crochets peuvent engager des crochets de fixateurs à crochets après que le film a été fixé par la résine refroidie au matériau apte à être engagé par crochets.

5. Procédé selon l'une quelconque des revendications précédentes, dans lequel le premier polymère est le polypropylène.

6. Procédé selon l'une quelconque des revendications précédentes, dans lequel le film (122) et le matériau (124) apte à être engagé par crochets sont essentiellement constitués de polypropylène.

7. Procédé selon l'une quelconque des revendications précédentes, dans lequel la résine fondue (126) est constituée d'au moins environ 90 pour cent en poids de polypropylène.

8. Procédé selon l'une quelconque des revendications précédentes, dans lequel la résine fondue (126) comprend en outre du polyéthylène.

9. Procédé selon l'une quelconque des revendications précédentes, dans lequel le matériau (124) apte à être engagé par crochets comprend un substrat de polypropylène non tissé à raison de 17 grammes par mètre carré, traversé par des fibres discontinues en forme de boucle.

10. Procédé selon l'une quelconque des revendications 1 à 8, dans lequel le matériau (124) apte à être engagé par crochets comprend un substrat de polypropylène SMS de 30 grammes par mètre carré, traversé par des fibres discontinues en forme de boucle.

11. Procédé selon l'une quelconque des revendications précédentes, dans lequel la résine fondue (126) introduite dans l'interstice (108) présente une épaisseur d'environ 0,0254 mm (un mil), le film (122) présente une épaisseur d'environ 0,0762 mm (trois mils) et le matériau (124) apte à être engagé par crochets présente un poids d'environ 44,1 g/m² à environ 67,8 g/m² (environ 1,3 à environ 2,0 osy ("ounce per square inch" - once par pouce carré).

12. Procédé selon l'une quelconque des revendications précédentes, dans lequel le matériau (124) apte à être engagé par crochets présente un poids de base d'environ 44,1 g/m² à environ 54,2 g/m² (environ 1,3 à environ 1,6 osy).

13. Procédé selon l'une quelconque des revendications précédentes, dans lequel la résine fondue (26) présente une température d'au moins environ 260 degrés Celsius (environ 500 degrés Fahrenheit) lorsqu'elle est introduite dans l'interstice (108).

14. Procédé selon l'une quelconque des revendications précédentes, dans lequel la résine fondue (126) présente une température d'environ 287,8 degrés Celsius à environ 315,6 degrés Celsius (d'environ 550 degrés Fahrenheit à environ 600 degrés Fahrenheit) lorsqu'elle est introduite dans l'interstice (108).

15. Procédé selon l'une quelconque des revendications précédentes, dans lequel la résine fondue (126) présente une température d'environ 304,4 degrés Celsius (environ 580 degrés Fahrenheit) lorsqu'elle est introduite dans l'interstice (108).

16. Procédé selon l'une quelconque des revendications précédentes, dans lequel le film (122) est un film coulé.

17. Procédé selon l'une quelconque des revendications précédentes, dans lequel la résine fondue (126) est introduite dans l'interstice (108) de telle sorte que la résine fondue (126) entre en contact avec le film (122) et le matériau (124) apte à être engagé par crochets essentiellement en même temps.

18. Procédé selon l'une quelconque des revendications précédentes, dans lequel la résine fondue (126) est introduite dans l'interstice (108) en appliquant la résine fondue (126) sur le matériau (124) apte à être engagé par crochets et en faisant tourner le premier et le deuxième cylindre (102, 104) de telle sorte qu'ils transportent l'interstice le film (122), le matériau (124) apte à être engagé par crochets et la résine fondue (126) dans.

19. Procédé selon l'une quelconque des revendications 1 à 17, dans lequel la résine fondue (126) est introduite dans l'interstice (108) en appliquant la résine fondue (126) sur le film (122) et en faisant tourner le premier et le deuxième cylindre (102, 104) de telle sorte qu'ils transportent dans l'interstice (108) le film (122), le matériau (124) apte à être engagé par crochets et la résine fondue (126).

20. Procédé selon l'une quelconque des revendications précédentes, dans lequel le premier et/ou le deuxième cylindre (102, 104) sont refroidis de manière à renforcer le refroidissement de la résine fondue (126).

21. Procédé selon l'une quelconque des revendications précédentes, dans lequel le premier et le deuxième rouleau (102, 104) sont refroidis.

22. Procédé selon l'une quelconque des revendications précédentes, comprenant en outre le gaufrage du matériau composite.

23. Procédé selon l'une quelconque des revendications précédentes, dans lequel le gaufrage du matériau composite comprend les étapes qui consistent à faire passer le matériau composite entre un cylindre de gaufrage (152) et un cylindre de dos (154), le cylindre de gaufrage (152) présentant plusieurs éléments en relief (158) qui compriment le matériau composite entre le cylindre de gaufrage (152) et le cylindre de dos (154).

24. Procédé selon la revendication 23, dans lequel le cylindre de gaufrage (152) est un cylindre chauffé.
